# EUROPÄISCHE PATENTSCHRIFT

(11) **EP 3 007 764 B1**
(45) Veröffentlichungstag und Bekanntmachung des Hinweises auf die Patenterteilung: **01.08.2018**
(21) Anmeldenummer: 14730506.4
(22) Anmeldetag: 12.06.2014
(51) Int. Cl.: A61N 1/375

(54) **HERZSCHRITTMACHERSYSTEM MIT EINER HALTEVORRICHTUNG**
CARDIAC PACEMAKER SYSTEM COMPRISING A MOUNTING ARRANGEMENT
SYSTÈME DE STIMULATEUR CARDIAQUE MUNI D'UN DISPOSITIF DE RETENUE

(30) Priorität: 12.06.2013 EP 13002991
(43) Veröffentlichungstag der Anmeldung: 20.04.2016
(73) Patentinhaber: Gutersohn, Achim, 49377 Vechta (DE)
(72) Erfinder: Gutersohn, Achim, 49377 Vechta (DE)
(74) Vertreter: Hauck Patentanwaltspartnerschaft mbB
(86) Internationale Anmeldenummer: PCT/EP2014/062224
(87) Internationale Veröffentlichungsnummer: WO 2014/198822

(56) Entgegenhaltungen:
- EP-A2- 0 306 443
- US-A1- 2008 077 219
- US-A1- 2008 288 039
- US-A1- 2012 197 349
- COHEN ET AL: "Reference values for normal adult transesophageal echocardiographic measurements", JOURNAL OF THE AMERICAN SOCIETY OF ECHOCARDIOGRAPHY, MOSBY-YEAR BOOK, INC. ST. LOUIS, MO, US, Bd. 8, Nr. 3, 1. Mai 1995 (1995-05-01), Seiten 221-230, XP005316816, ISSN: 0894-7317, DOI: 10.1016/S0894-7317(05)80031-8

## Beschreibung

Die Erfindung betrifft ein Herzschrittmachersystem mit einem Steuergerät, das eine Energiequelle, eine elektronische Steuerung und eine erste elektrische Kontaktfläche aufweist sowie eine erste Sonde zur Anordnung in oder an einer ersten Herzkammer.

Derartige Herzschrittmachersysteme sind seit Jahrzehnten bekannt. Das Steuergerät enthält einen Impulsgeber und stimuliert über die Sonde einen Herzmuskel mithilfe von elektrischen Impulsen, um diesen zu Kontraktionen anzuregen. Bei bekannten Herzschrittmachersystemen können die Sonden eine Doppelfunktion aufweisen. Einerseits dienen sie zum Abgeben der elektrischen Impulse, andererseits leiten sie dem Steuergerät elektrische Potentiale aus dem Herzen zu, um dessen Funktion überwachen und beispielsweise auf Unregelmäßigkeiten reagieren zu können. Ebenfalls bekannt sind Zwei- oder Dreikammerherzschrittmachersysteme, bei denen mehrere Sonden in unterschiedlichen Herzkammern angeordnet werden. Auf diese Weise kann beispielsweise mithilfe einer getriggerten Stimulation eine Leitungsstörung zwischen Vorhof und Kammer überbrückt und erfolgreich behandelt werden. Moderne Herzschrittmachersysteme werden zur Behandlung der unterschiedlichsten Störungen eingesetzt, insbesondere für die kardiale Resynchronisationstherapie (CRT, cardiac resynchronization therapy).

Bei den meisten im Einsatz befindlichen Herzschrittmachern wird das Steuergerät subkutan in das Fettgewebe implantiert, häufig oberhalb einer Brust unterhalb des Schlüsselbeins oder auch unterhalb eines großen Brustmuskels. Der oder die Sonden werden in der Regel transvenös zu der oder den betreffenden Herzkammern geführt, was die Verwendung relativ langer elektrischer Leitungen erfordert.

Aus der Druckschrift EP 0 306 443 A2 ist ein Koaxialstecker für einen solchen Herzschrittmacher mit subkutan zu implantierendem Steuergerät bekannt geworden.

Ein Ende des Koaxialsteckers ist zum Einstecken in das Steuergerät vorgesehen und weist zwei Kontaktflächen auf. Aus dem anderen Ende des Koaxialsteckers sind zwei mit diesen Kontaktflächen verbundene Elektroden herausgeführt. Weder das Steuergerät noch der damit verbundene Koaxialstecker sind zum Einsetzen in ein Blutgefäß geeignet.

Ebenfalls bekannt, beispielsweise aus der Druckschrift EP 2 471 447 A1, sind sogenannte leitungslose Herzschrittmachersysteme (leadless pacemaker). Diese weisen ein miniaturisiertes Steuergerät auf, das unmittelbar in oder an der betreffenden Herzkammer angeordnet wird. Zur Befestigung weisen die aus der Druckschrift bekannten Systeme ein spiralförmiges Verankerungselement auf, das unmittelbar im Herzmuskel verankert werden kann. Derartige Systeme sind zunächst nur zur Behandlung einer einzigen Herzkammer geeignet. Sollen mehrere Herzkammern, beispielsweise rechter Vorhof und rechter Ventrikel, in die Therapie einbezogen werden, können mehrere derartige Schrittmachersysteme implantiert werden, die untereinander kommunizieren müssen. Eine hierfür grundsätzlich mögliche Funkverbindung ist wegen des relativ hohen Energieverbrauchs nachteilig. Die genannte Druckschrift schlägt daher eine Kommunikation zwischen mehreren derartigen Schrittmachersystemen durch Leitung elektrischer Signale durch das Gewebe vor. Dennoch stellt der Energieverbrauch derartiger Herzschrittmachersysteme ein Problem dar, da wegen der erforderlichen Miniaturisierung nur sehr kleine Batterien eingesetzt werden können. Wird ein Batteriewechsel erforderlich, erfordert dies einen aufwendigen medizinischen Eingriff zur Explantation des gesamten Systems.

Zum Implantieren und Explantieren bekannter leitungsloser Herzschrittmachersysteme werden spezielle Implantiersysteme eingesetzt, wie beispielsweise in der Druckschrift WO 2012/082735 A1 beschrieben. Wegen der Verankerung im Herzmuskel besteht allerdings auch bei Verwendung derartiger Systeme stets das Risiko einer Schädigung des Herzmuskels, sowohl bei der Implantation als auch bei der Explantation.

Aus der Druckschrift US 2008/0288039 A1 ist ein in ein Blutgefäß implantierbares Herzschrittmachersystem bekannt geworden, das ein Steuergerät und eine elektrische Leitung aufweist. Zur Implantation kann ein Führungsdraht durch eine Durchgangsöffnung im Steuergerät hindurch in ein Lumen der elektrischen Leitung eingeführt werden, so dass das System in einer langgestreckten Anordnung an seinen Bestimmungsort vorgeschoben werden kann. Nach dem Entfernen des Führungsdrahts wickelt sich die elektrische Leitung spiralförmig auf und legt sich an eine Gefäßwand an, wodurch das Herzschrittmachersystem in dem Gefäß fixiert wird. Um das Herzschrittmachersystem wieder aus dem Gefäß zu entfernen, kann der Führungsdraht wieder durch die Durchgangsöffnung des Steuergeräts hindurch in das Lumen der elektrischen Leitung eingeführt werden, so dass die elektrische Leitung wieder ihre gestreckte Form annimmt.

Aus der Druckschrift US 2008/0077219 A1 ist ein Herzschrittmachersystem mit den Merkmalen des Oberbegriffs des Anspruchs 1 bekannt geworden.

Davon ausgehend ist es die Aufgabe der Erfindung, ein Herzschrittmachersystem mit einem Steuergerät und einer Sonde zur Verfügung zu stellen, das insbesondere für einen Batteriewechsel einen einfacheren Austausch des Steuergeräts ermöglicht und dadurch auch über längere Zeiträume einen zuverlässigen Betrieb vereinfacht.

Diese Aufgabe wird gelöst durch das Herzschrittmachersystem mit den Merkmalen des Anspruchs 1. Vorteilhafte Ausgestaltungen sind in den Unteransprüchen angegeben.

Die Energiequelle des Steuergeräts kann insbesondere eine Batterie sein. Die elektronische Steuerung ist dazu eingerichtet, über die erste elektrische Kontaktfläche Impulse zur Stimulation eines Herzmuskels abzugeben. Diese werden über die erste elektrische Kontaktfläche und das erste elektrische Kontaktelement an die erste Sonde geleitet. Gegebenenfalls ist die elektronische Steuerung zusätzlich dazu ausgebildet, von der ersten Sonde abgeleitete Signale auszuwerten. Hierzu kann die erste Sonde eine oder mehrere elektrische Leitungen aufweisen. Darüber hinaus kann die elektronische Steuerung eine Kommunikationseinrichtung zum Austausch von Daten mit einem externen Gerät aufweisen.

Die erste Sonde ist zur Anordnung in oder an einer ersten Herzkammer vorgesehen. Sie kann hierzu insbesondere über eine Vene an einen Herzmuskel der ersten Herzkammer herangeführt sein. Grundsätzlich ist auch eine Anordnung der ersten Sonde innerhalb der ersten Herzkammer möglich. Auch in diesem Fall kann die erste Sonde an dem betreffenden Herzmuskel angeordnet sein. Die erste Herzkammer kann insbesondere der linke Ventrikel sein.

Das erfindungsgemäße Herzschrittmachersystem weist eine in ein Blutgefäß einsetzbare Haltevorrichtung für das Steuergerät auf. Die Haltevorrichtung ist dabei insbesondere hinsichtlich ihrer Abmessungen an die Abmessungen des Blutgefäßes, in das die Vorrichtung eingesetzt werden soll, angepasst. Sie wird in das betreffende Blutgefäß eingesetzt oder implantiert und kann über einen längeren Zeitraum darin verbleiben, insbesondere auch bei einem Austausch des Steuergeräts. Dass die Haltevorrichtung in das Blutgefäß einsetzbar ist, bedeutet, dass sie dazu ausgebildet ist, eine dauerhafte Fixierung und/oder Verankerung des Herzschrittmachersystems in dem Blutgefäß zu bewirken. Sie beeinträchtigt dabei den Blutfluss in dem Blutgefäß nicht mehr als vertretbar. Zum Beispiel können die Abmessungen des Herzschrittmachersystem so an ein Ziel-Blutgefäß angepasst sein, dass ein offener Querschnitt des Blutgefäßes durch das Herzschrittmachersystem um maximal 75% verringert wird, vorzugsweise nur um maximal 50% oder um maximal 25%.

Die Haltevorrichtung weist ein Halteelement zur lösbaren Befestigung des Steuergeräts an der Haltevorrichtung auf. Hierzu kann das Steuergerät korrespondierende Halteabschnitte aufweisen, die mit dem Halteelement zusammenwirken. Insbesondere kann das Steuergerät in die Haltevorrichtung eingesetzt werden oder darin einrasten. In dem an der Haltevorrichtung befestigten Zustand befindet sich das Steuergerät dann in einer relativ zu der Haltevorrichtung fest vorgegebenen Position.

Die Haltevorrichtung weist ein erstes elektrisches Kontaktelement auf, das elektrisch mit der ersten Sonde verbunden ist. Somit ist die erste Sonde bzw. ein Teil davon in die Haltevorrichtung integriert. Bei an der Haltevorrichtung befestigtem Steuergerät steht das erste elektrische Kontaktelement im elektrischen Kontakt mit der ersten Kontaktfläche. Dadurch wird beim Befestigen des Steuergeräts an der Haltevorrichtung automatisch ein elektrischer Kontakt zwischen dem Steuergerät und der ersten Sonde hergestellt. Es ist daher nicht erforderlich, bei einem Austausch des Steuergeräts die Positionierung der ersten Sonde in oder an der ersten Herzkammer zu verändern oder anzutasten. Dennoch befindet sich das Steuergerät viel näher als ein herkömmlicher, subkutan implantierter Herzschrittmacher an der ersten Herzkammer. Die Verbindungsleitungen zwischen Sonde und Steuergerät können daher wesentlich kürzer ausgeführt werden, was zu einem problemlosen Betrieb des Herzschrittmachersystems auch über längere Zeiträume beiträgt.

Die Erfindung vereinfacht den zum Austausch des Steuergeräts erforderlichen medizinischen Eingriff auf zweierlei Weise. Zum einen befindet sich das Steuergerät gegebenenfalls in einem relativ großen Blutgefäß, sodass es viel einfacher zugänglich ist als ein unmittelbar in oder an einer Herzkammer angeordnetes, leitungsloses Herzschrittmachersystem. Zum anderen ermöglicht das Halteelement der Haltevorrichtung eine lösbare Befestigung des Steuergeräts, sodass zum Einsetzen und Entnehmen des Steuergeräts eine einfache und zuverlässige, gegebenenfalls für einen mehrfachen Wechsel ausgelegte Schnittstelle vorhanden ist. Insbesondere muss das Gewebe des Patienten in der Umgebung des Herzschrittmachersystems für einen Austausch des Steuergeräts nicht angetastet werden.

In einer Ausgestaltung ist das Steuergerät in die Haltevorrichtung einsetzbar. Das Steuergerät befindet sich demnach ganz oder teilweise innerhalb der Haltevorrichtung und/oder wird von der Haltevorrichtung ganz oder teilweise umschlossen. Dies ergibt eine besonders kompakte Anordnung.

In einer Ausgestaltung hat das Herzschrittmachersystem eine zweite Sonde zur Anordnung in oder an einer zweiten Herzkammer, wobei das Steuergerät eine zweite elektrische Kontaktfläche aufweist und die Haltevorrichtung ein zweites elektrisches Kontaktelement aufweist, das elektrisch mit der zweiten Sonde verbunden ist und an das bei an der Haltevorrichtung befestigtem Steuergerät im elektrischen Kontakt mit der zweiten Kontaktfläche steht. Auf diese Weise kann das Steuergerät ebenso einfach wie vorstehend für die erste Sonde beschrieben beim Befestigen an der Haltevorrichtung automatisch mit der zweiten Sonde verbunden werden. Dies ermöglicht einen Zweikammerbetrieb des Herzschrittmachersystems, wobei es gegenüber herkömmlichen, subkutan implantierten Steuergeräten nicht zu einem erhöhten Energieverbrauch kommt, da eine Kommunikation unterschiedlicher Steuergeräte über eine Funkverbindung oder auf sonstige Art und Weise nicht erforderlich ist. Es versteht sich, dass das Herzschrittmachersystem wahlweise, insbesondere für einen Dreikammerbetrieb, mit einer entsprechend ausgebildeten dritten Sonde zur Anordnung in oder an einer dritten Herzkammer ausgerüstet sein kann. In diesem Fall kann das Steuergerät eine dritte elektrische Kontaktfläche aufweisen und die Haltevorrichtung ein drittes elektrisches Kontaktelement, das elektrisch mit der dritten Sonde verbunden ist und das bei an der Haltevorrichtung befestigtem Steuergerät im elektrischen Kontakt mit der dritten Kontaktfläche steht. Die zweite Herzkammer kann insbesondere das linke Atrium sein, die dritte Herzkammer insbesondere der rechte Ventrikel.

In einer Ausgestaltung ist die Haltevorrichtung zum Einsetzen in einen Koronarvenensinus ausgebildet. Der Koronarvenensinus ist wegen seines relativ großen Durchmessers und seiner Anordnung in unmittelbarer Nähe zu den zu behandelnden Herzkammern zum Einsetzen der erfindungsgemäßen Haltevorrichtung besonders geeignet. Ein weiterer Vorteil besteht darin, dass über die mit dem Koronarvenensinus verbundenen weiteren Blutgefäße Sonden zu mehreren zu behandelnden Herzkammern geführt werden können.

In einer Ausgestaltung weist die Haltevorrichtung einen röhrenförmigen Körper auf, der im in das Blutgefäß eingesetzten Zustand an einer Gefäßwand anliegt. Auf diese Weise kann die Haltevorrichtung dauerhaft am gewünschten Ort in dem Blutgefäß angeordnet werden.

In einer Ausgestaltung weist der röhrenförmige Körper ein Geflecht, Gewebe oder Gerüst aus Draht oder Blech auf. Insbesondere kann der röhrenförmige Körper ein Stent sein. Elastizität und Festigkeit des röhrenförmigen Körpers können so gewählt sein, dass ein problemloses Einsetzen der Haltevorrichtung in das Blutgefäß an der gewünschten Stelle möglich ist und der röhrenförmige Körper nach dem Einsetzen zuverlässig Halt an der Gefäßwand findet. Der Draht oder das Blech können grundsätzlich aus einem beliebigen biokompatiblen Metall oder auch aus einem Kunststoffmaterial bestehen. Besonders geeignet ist Nitinol. Die Drähte können an Kreuzungspunkten miteinander verbunden sein, beispielsweise durch Klammern oder durch Verschweißen. Weist der röhrenförmige Körper ein Blech auf, kann dieses mit einer Vielzahl von Öffnungen versehen sein und beispielsweise vor oder nach dem Formen in die röhrenförmige Gestalt lasergeschnitten werden.

In einer Ausgestaltung ist das Halteelement so angeordnet, dass das daran befestigte Steuergerät im Inneren des röhrenförmigen Körpers angeordnet ist, sodass es allseitig einen Abstand zu dem röhrenförmigen Körper aufweist. Es verbleibt also ein ringförmiger Freiraum um das Steuergerät herum, was einen ausreichenden Blutfluss durch das Blutgefäß ermöglicht und Strömungsturbulenzen im Bereich der Gefäßwand, die langfristig zu Problemen führen können, weitgehend vermeidet.

In einer Ausgestaltung weist die Haltevorrichtung ein erstes Ende, ein zweites Ende und einen mittleren Abschnitt auf, wobei das Halteelement an dem mittleren Abschnitt angeordnet ist und die erste Sonde eine erste elektrische Leitung aufweist, die am ersten Ende aus der Haltevorrichtung herausgeführt ist. Der mittlere Abschnitt ist zwischen dem ersten Ende und dem zweiten Ende angeordnet. Ein Teil der ersten elektrischen Leitung ist also in die Haltevorrichtung integriert. Dieser Teil kann insbesondere von einem gegebenenfalls isolierten Draht der Haltevorrichtung selbst gebildet sein oder an der Haltevorrichtung befestigt. In beiden Fällen wird eine Bewegung der ersten elektrischen Leitung im Bereich der Haltevorrichtung vermieden, was einem Verschleiß der ersten elektrischen Leitung in diesem sensiblen Bereich entgegenwirkt. Die erste elektrische Leitung kann eine Länge im Bereich von 3 mm bis 150 mm aufweisen, insbesondere im Bereich von 10 mm bis 100 mm.

In einer Ausgestaltung weist die zweite Sonde eine zweite elektrische Leitung auf, die am zweiten Ende aus der Haltevorrichtung herausgeführt ist. Insbesondere kann das zweite Ende dem ersten Ende gegenüberliegen. Dadurch ergeben sich zunächst für die zweite elektrische Leitung die im Zusammenhang mit der ersten elektrischen Leitung vorstehend erläuterten Vorteile. Zum anderen wird die Positionierung der beiden Sonden dadurch vereinfacht, dass die außerhalb der Haltevorrichtung anzuordnenden elektrischen Leitungen bereits an einer optimalen Position aus der Haltevorrichtung herausgeführt sind. Vermieden wird, dass erste und zweite elektrische Leitung aneinander reiben oder sich auf sonstige Weise gegenseitig nachteilig beeinflussen. Die zweite elektrische Leitung kann eine Länge im Bereich von 3 mm bis 150 mm aufweisen, insbesondere im Bereich von 10 mm bis 100 mm.

In einer Ausgestaltung weist das Haltelement einen Ring und mindestens eine von dem Ring in dessen Mitte weisende Querstrebe auf, wobei das Steuergerät an der mindestens einen Querstrebe befestigt wird. Der Außendurchmesser des Rings kann insbesondere ungefähr dem Innendurchmesser des zum Einsetzen der Haltevorrichtung vorgesehenen Blutgefäßes entsprechen. Der Ring kann insbesondere in Umfangsrichtung des Blutgefäßes an der Haltevorrichtung angeordnet sein. Der Ring kann fest mit dem röhrenförmigen Körper verbunden sein oder nachträglich in diesen eingesetzt und daran befestigt werden. Der Ring kann aus einem biokompatiblen Material bestehen oder ein solches Material aufweisen, beispielsweise Titan, Nitinol oder ein geeignetes Kunststoffmaterial. Die Querstrebe kann einteilig mit dem Ring ausgebildet oder an dem Ring befestigt sein. Sie weist in die Mitte des Rings, erstreckt sich jedoch nicht unbedingt bis zur Mitte hin.

Möglich ist allerdings auch eine sich über den gesamten Durchmesser des Rings erstreckende Querstrebe. Die Querstrebe bietet die Möglichkeit, das Steuergerät an einer optimalen Position innerhalb des Rings bzw. der Haltevorrichtung zu befestigen.

In einer Ausgestaltung sind zwei zur Mitte des Rings weisende Querstreben vorhanden, die in einem Abstand voneinander an einander gegenüberliegenden Positionen enden und dort jeweils eines der Kontaktelemente aufweisen. In diesem Fall kann das Steuergerät zwischen den freien Enden der beiden Querstreben eingesetzt werden. Die Querstreben können dann gleichzeitig die mechanische Befestigung des Steuergeräts bewerkstelligen und den elektrischen Kontakt zu den beiden Sonden herstellen.

In einer Ausgestaltung weist das Steuergerät ein zylindrisches, kapselförmiges Gehäuse auf. Der Durchmesser des Gehäuses kann geringer sein als der Außendurchmesser der Haltevorrichtung, insbesondere kann er weniger als 60% davon oder sogar weniger als 50% oder weniger als 40% davon betragen. Das Steuergerät kann eine langgestreckte Form aufweisen. Die beiden Enden des Steuergeräts können halbkugelförmig abgerundet sein oder leicht spitz zulaufen. Insbesondere kann das gesamte Gehäuse eine hydrodynamische Stromlinienform aufweisen. Das Gehäuse kann aus einem biokompatiblen Material bestehen, beispielsweise wie im Zusammenhang mit dem Ring bereits erläutert.

In einer Ausgestaltung weist das Steuergerät eine Öffnung zum Hindurchführen eines Führungsdrahts auf. Mithilfe des Führungsdrahts kann das Steuergerät durch ein Blutgefäß bis an den Ort der Haltevorrichtung vorgeschoben werden. Die Öffnung kann hierzu insbesondere eine Durchgangsöffnung sein. Sie kann insbesondere in Längsrichtung des Gehäuses verlaufen. Sie kann entlang einer Mittellängsachse des Gehäuses angeordnet sein oder bevorzugt nahe einem äußeren Rand des Gehäuses.

In einer Ausgestaltung weist das Steuergerät Führungsmittel auf, die mit dem Halteelement zusammenwirken, um das Steuergerät beim Anordnen an der Haltevorrichtung in eine definierte Befestigungsposition relativ zu der Haltevorrichtung zu führen. Die Führungsmittel können beispielsweise Führungsflächen, Führungsrippen, Führungsnuten oder Führungsstifte sein. Insbesondere können die Führungsmittel eine Schrägfläche aufweisen, die beim Annähern des Steuergeräts an das Halteelement eine Rotation des Steuergeräts um seine Längsachse bewirken. Das Halteelement kann ebenfalls Führungsflächen aufweisen, die mit dem Führungsmittel des Steuergeräts zusammenwirken.

In einer Ausgestaltung liegen die Kontaktflächen an dem Steuergerät einander gegenüber. Dies ist einerseits für eine elektrische Isolation der Kontaktflächen voneinander vorteilhaft. Andererseits ermöglicht es eine besonders zuverlässige elektrische Kontaktierung über die beiden elektrischen Kontaktelemente, weil von diesen auf die Kontaktflächen ausgeübte Kräfte beide elektrischen Kontakte gleichermaßen begünstigen.

Die Erfindung wird nachfolgend anhand eines in Figuren dargestellten Ausführungsbeispiels näher erläutert. Es zeigen:
- Fig. 1: ein erfindungsgemäßes Herzschrittmachersystem in einer perspektivischen, teilweise schematisch vereinfachten Darstellung,
- Fig. 2: einen Teil der Haltevorrichtung und das Steuergerät aus Fig. 1 in einer weiteren perspektivischen Darstellung,
- Fig. 3: eine weitere perspektivische Ansicht der in Fig. 2 dargestellten Elemente des Herzschrittmachersystems.

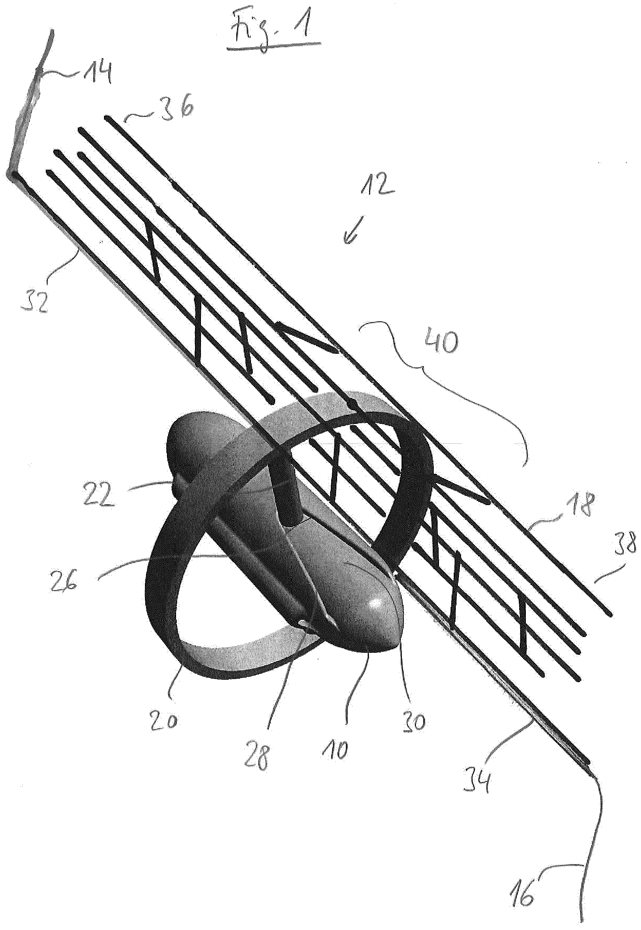

Das Herzschrittmachersystem aus Fig. 1 hat ein Steuergerät 10, eine Haltevorrichtung 12, eine erste Sonde 14 und eine zweite Sonde 16. Die Haltevorrichtung 12 weist einen käfigartigen, röhrenförmigen Körper 18 aus Nitinoldrähten auf. Dieser röhrenförmige Körper 18 ist in der Fig. 1 nur teilweise dargestellt. In Wirklichkeit ist der röhrenförmige Körper 18 im Querschnitt etwa kreisförmig und umgibt das Steuergerät 10 vollständig. Der röhrenförmige Körper 18 weist eine gewisse Elastizität auf und ist dazu ausgebildet, an der Innenseite einer Gefäßwand eines Blutgefäßes, in das die Haltevorrichtung eingesetzt wird, anzuliegen.

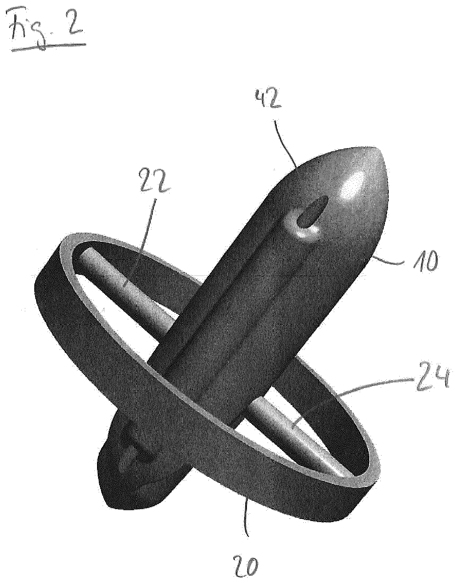

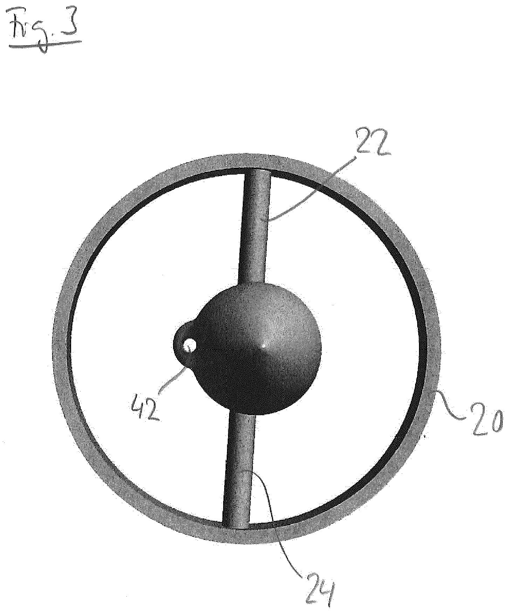

Die Haltevorrichtung 12 weist außerdem einen Ring 20 auf, der an der Innenseite des röhrenförmigen Körpers 18 befestigt ist. Ausgehend von der Innenseite des Rings 20 erstreckt sich eine erste Querstrebe 22 in Richtung zu der Mitte des Rings 20 hin. Diese endet in einem Abstand vom Mittelpunkt des Rings 20. Eine zweite Querstrebe 24 (siehe Fig. 2 und 3) ist in der Fig. 1 von dem Steuergerät 10 verdeckt.

Der Ring 20, die erste Querstrebe 22 und die zweite Querstrebe 24 bilden gemeinsam ein Halteelement für das Steuergerät 10, an dem das Steuergerät 10 lösbar befestigt ist. Hierzu weisen die beiden Querstreben 22, 24 jeweils an ihrem freien Ende einen Befestigungsabschnitt 26 auf. Die Befestigungsabschnitte 26 weisen zwei gegenüberliegende, unter einem Winkel zueinander angeordnete Schrägflächen auf, die beim Einsetzen des Steuergeräts 10 in die Haltevorrichtung 12 mit ebenfalls schräg angeordneten Führungsflächen 28 am Steuergerät 10 zusammenwirken. Jeweils zwei dieser Führungsflächen 28 sind auf jeder Seite des Steuergeräts 10 ausgebildet und begrenzen gemeinsam eine V-förmige Vertiefung 30 in der im Übrigen im Wesentlichen kreiszylindrischen Oberfläche des Gehäuses des Steuergeräts 10.

Teilweise in die Haltevorrichtung 12 integriert sind die erste Sonde 14 und die zweite Sonde 16. Die erste Sonde 14 weist eine erste elektrische Leitung 32 und die zweite Sonde 16 weist eine zweite elektrische Leitung 34 auf. Abschnitte dieser beiden Leitungen 32, 34 sind an einem Nitinoldraht des röhrenförmigen Körpers 18 entlanggeführt und daran befestigt. Sie weisen darüber hinaus jeweils einen Abschnitt auf, der über den röhrenförmigen Körper 18 übersteht. An den Enden dieser überstehenden Abschnitte werden die Sonden 14, 16 in elektrischen Kontakt mit den zu stimulierenden bzw. abzutastenden Herzmuskeln gebracht. Die Sonden 14, 16 sind in der Fig. 1 nur schematisch vereinfacht dargestellt.

Die erste elektrische Leitung 32 führt von einem ersten Ende 36 des röhrenförmigen Körpers 18 zu einem mittleren Abschnitt 40 davon und verläuft von dort weiter über den Ring 20 in die erste Querstrebe 22 hinein. Im Bereich des Befestigungsabschnitts 26 endet die erste elektrische Leitung 32 an einem nicht dargestellten ersten elektrischen Kontaktelement. Dieses steht bei an der Haltevorrichtung 12 befestigtem Steuergerät 10 im elektrischen Kontakt mit einer ebenfalls nicht im Einzelnen erkennbaren ersten Kontaktfläche des Steuergeräts 10. Entsprechend ist die zweite elektrische Leitung 34 von einem zweiten Ende 38, das dem ersten Ende 36 des röhrenförmigen Körpers 18 gegenüberliegt, hin zu dem mittleren Abschnitt 40 geführt und verläuft von dort über den Ring 20 und die zweite Querstrebe 24 bis hin zu einer zweiten Kontaktfläche, die an einem Befestigungsabschnitt 26 am freien Ende der zweiten Querstrebe 24 angeordnet ist. Sie gelangt bei in die Haltevorrichtung 12 eingesetztem Steuergerät 10 in elektrischen Kontakt mit einer am Steuergerät 10 ausgebildeten, zweiten Kontaktfläche.

Man erkennt in der Fig. 1, dass das Steuergerät 10 allseitig einen Abstand zu dem röhrenförmigen Körper 18 und dem Ring 20 aufweist. Durch den Freiraum zwischen dem Ring 20 und dem Steuergerät 10 ist eine ausreichende Blutzirkulation durch ein Blutgefäß, in das die Haltevorrichtung 12 eingesetzt ist, gewährleistet.

Weitere Einzelheiten des Steuergeräts 10 sind besser in den Fig. 2 und 3 erkennbar, in denen der röhrenförmige Körper 18 mit der ersten und zweiten Sonde 14, 16 nicht gezeigt ist. Man erkennt in der Fig. 2, dass das Steuergerät 10 ein zylindrisches, kapselförmiges Gehäuse mit einer langgestreckten Form aufweist. Das vordere und hintere Ende dieses Gehäuses sind leicht spitz auslaufend, stromlinienförmig abgerundet ausgebildet. An einer Seite des Gehäuses befindet sich eine Öffnung 42, die in Längsrichtung des Steuergeräts 10 durch dessen Gehäuse hindurch verläuft und durch die ein Führungsdraht hindurchgeführt werden kann. Die Öffnung 42 befindet sich in einem seitlichen Abstand von einer Mittellängsachse des Steuergeräts 10.

Ebenfalls gut in Fig. 2 zu erkennen ist die zweite Querstrebe 24 und dass das Steuergerät 10 zwischen der ersten Querstrebe 22 und der zweiten Querstrebe 24 gehalten ist.

In der Fig. 3 ist der Blick auf eines der Enden des Steuergeräts 10 gerichtet. Man erkennt ebenfalls die Halterung des Steuergeräts 10 zwischen der ersten Querstrebe 22 und der zweiten Querstrebe 24 und die durchgängige Öffnung 42 an einer Seite des Steuergeräts 10.

### Liste der verwendeten Bezugszeichen:

- 10: Steuergerät
- 12: Haltevorrichtung
- 14: Erste Sonde
- 16: Zweite Sonde
- 18: Röhrenförmiger Körper
- 20: Ring
- 22: Erste Quersterbe
- 24: Zweite Querstrebe
- 26: Befestigungsabschnitt
- 28: Führungsflächen
- 30: Vertiefung
- 32: Erste elektrische Leitung
- 34: Zweite elektrische Leitung
- 36: Erstes Ende
- 38: Zweites Ende
- 40: Mittlerer Abschnitt
- 42: Öffnung

## Patentansprüche

1. Herzschrittmachersystem mit
• einem Steuergerät (10), das eine Energiequelle, eine elektronische Steuerung und eine erste elektrische Kontaktfläche aufweist,
• einer ersten Sonde (14) zur Anordnung in oder an einer ersten Herzkammer und
• einer in ein Blutgefäß einsetzbaren Haltevorrichtung (12), in die das Steuergerät (10) einsetzbar ist und
• die ein Halteelement zur lösbaren Befestigung des Steuergeräts (10) an der Haltevorrichtung (12) aufweist, **dadurch gekennzeichnet, dass**
• die Haltevorrichtung (12) ein erstes elektrisches Kontaktelement aufweist, das elektrisch mit der ersten Sonde (14) verbunden ist und das bei an der Haltevorrichtung (12) befestigtem Steuergerät (10) im elektrischen Kontakt mit der ersten Kontaktfläche steht.

2. Herzschrittmachersystem nach Anspruch 1, **gekennzeichnet durch** eine zweite Sonde (16) zur Anordnung in oder an einer zweiten Herzkammer, wobei das Steuergerät (10) eine zweite elektrische Kontaktfläche aufweist und die Haltevorrichtung (12) ein zweites elektrisches Kontaktelement aufweist, das elektrisch mit der zweiten Sonde (16) verbunden ist und das bei an der Haltevorrichtung (12) befestigtem Steuergerät (10) im elektrischen Kontakt mit der zweiten Kontaktfläche steht.

3. Herzschrittmachersystem nach Anspruch 1 oder 2, **dadurch gekennzeichnet, dass** die Haltevorrichtung (12) zum Einsetzen in einen Koronarvenensinus ausgebildet ist.

4. Herzschrittmachersystem nach einem der Ansprüche 1 bis 3, **dadurch gekennzeichnet, dass** die Haltevorrichtung (12) einen röhrenförmigen Körper (18) aufweist, der im in das Blutgefäß eingesetzten Zustand an einer Gefäßwand anliegt.

5. Herzschrittmachersystem nach Anspruch 4, **dadurch gekennzeichnet, dass** der röhrenförmige Körper (18) ein Geflecht, Gewebe oder Gerüst aus Draht oder Blech aufweist.

6. Herzschrittmachersystem nach Anspruch 4 oder 5, **dadurch gekennzeichnet, dass** das Halteelement so angeordnet ist, dass das daran befestigte Steuergerät (10) im Inneren des röhrenförmigen Körpers (18) angeordnet ist, so dass es allseitig einen Abstand zu dem röhrenförmigen Körper (18) aufweist.

7. Herzschrittmachersystem nach einem der Ansprüche 1 bis 6, **dadurch gekennzeichnet, dass** das Steuergerät (10) ein zylindrisches, kapselförmiges Gehäuse aufweist.

8. Herzschrittmachersystem nach einem der Ansprüche 1 bis 7, **dadurch gekennzeichnet, dass** das Steuergerät (10) eine Öffnung (42) zum Hindurchführen eines Führungsdrahts aufweist.

9. Herzschrittmachersystem nach einem der Ansprüche 1 bis 8, **dadurch gekennzeichnet, dass** das Steuergerät (10) Führungsmittel aufweist, die mit dem Halteelement zusammenwirken, um das Steuergerät (10) beim Anordnen an der Haltevorrichtung (12) in eine definierte Befestigungsposition relativ zu der Haltevorrichtung (12) zu führen.

## Claims

1. A cardiac pacemaker system comprising
• a control unit (10) that has an energy source, an electronic control and a first electric contact surface,
• a first probe (14) for arranging in or on a first cardiac ventricle, and
• a mounting device (12) that can be inserted into a blood vessel, into which the control unit (10) can be inserted and
• that has a mounting element for releasably fastening the control unit (10) to the mounting device (12), **characterized in that**
• the mounting device (12) has a first electric contact element that is electrically connected to the first probe (14) and that is in electrical contact with the first contact surface when the control unit (10) is fastened to the mounting device (12).

2. The cardiac pacemaker system according to claim 1, **characterized by** a second probe (16) for arranging in or on a second cardiac ventricle, wherein the control unit (10) has a second electric contact surface and the mounting device (12) has a second electric contact element that is electrically connected to the second probe (16) and that is in electrical contact with the second contact surface when the control unit (10) is fastened to the mounting device (12).

3. The cardiac pacemaker system according to claim 1 or 2, **characterized in that** the mounting device (12) is designed for inserting into a coronary vein sinus.

4. The cardiac pacemaker system according to one of claims 1 to 3, **characterized in that** the mounting device (12) has a tubular body (18) that, in the inserted state in the blood vessel, abuts a vessel wall.

5. The cardiac pacemaker system according to claim 4, **characterized in that** the tubular body (18) has a mesh, weave or frame made of wire or sheet metal.

6. The cardiac pacemaker system according to claim 4 or 5, **characterized in that** the mounting element is arranged so that the control unit (10) fastened thereto is arranged on the inside of the tubular body (18) so that it has a spacing from the tubular body (18) on all sides.

7. The cardiac pacemaker system according to one of claims 1 to 6, **characterized in that** the control unit (10) has a cylindrical, capsule-shaped housing.

8. The cardiac pacemaker system according to one of claims 1 to 7, **characterized in that** the control unit (10) has an opening (42) for guiding a guide wire through.

9. The cardiac pacemaker system according to one of claims 1 to 8, **characterized in that** the control unit (10) has guide means that work together with the mounting element to guide the control unit (10) during the arranging on the mounting device (12) into a defined fastening position relative to the mounting device (12).

## Revendications

1. Système de stimulateur cardiaque comportant
• une unité de commande (10) qui dispose d'une source d'énergie, d'une commande électronique et d'une première surface de contact électrique,
• une première sonde (14) destinée à être disposée dans ou sur un premier ventricule cardiaque et
• un mécanisme de retenue (12) qui peut être inséré dans un vaisseau sanguin, dans lequel l'unité de commande (10) peut être insérée et
• qui dispose d'un élément de retenue afin de fixer de façon séparable l'unité de commande (10) sur le mécanisme de retenue (12), **caractérisé en ce que**
• le mécanisme de retenue (12) dispose d'un premier élément de contact électrique qui est électriquement raccordé à la première sonde (14) et qui est en contact électrique avec la première surface de contact si l'unité de commande (10) est fixée sur le mécanisme de retenue (12).

2. Système de stimulateur cardiaque selon la revendication 1, **caractérisé par** une seconde sonde (16) destinée à être disposée dans ou sur un second ventricule cardiaque, dans lequel l'unité de commande (10) dispose d'une seconde surface de contact électrique et le mécanisme de retenue (12) dispose d'un second élément de contact électrique qui est électriquement raccordé à la seconde sonde (16) et qui est en contact électrique avec la seconde surface de contact si l'unité de commande (10) est fixée sur le mécanisme de retenue (12).

3. Système de stimulateur cardiaque selon la revendication 1 ou 2, **caractérisé en ce que** le mécanisme de retenue (12) est conçu pour être inséré dans un sinus de veine coronaire.

4. Système de stimulateur cardiaque selon l'une des revendications 1 à 3, **caractérisé en ce que** le mécanisme de retenue (12) dispose d'un corps tubulaire (18) qui, dans l'état inséré dans le vaisseau sanguin, bute contre une paroi vasculaire.

5. Système de stimulateur cardiaque selon la revendication 4, **caractérisé en ce que** le corps tubulaire (18) dispose d'une maille, d'un tissage ou d'une trame constitué d'un métal en fil ou en feuille.

6. Système de stimulateur cardiaque selon la revendication 4 ou 5, **caractérisé en ce que** l'élément de retenue est disposé de manière à ce que l'unité de commande (10) fixée sur celui-ci soit disposée sur l'intérieur du corps tubulaire (18), de sorte qu'elle dispose d'un écartement par rapport au corps tubulaire (18) sur tous les côtés.

7. Système de stimulateur cardiaque selon l'une des revendications 1 à 6, **caractérisé en ce que** l'unité de commande (10) dispose d'un logement cylindrique en forme de capsule.

8. Système de stimulateur cardiaque selon l'une des revendications 1 à 7, **caractérisé en ce que** l'unité de commande (10) dispose d'une ouverture (42) afin de guider un fil-guide à travers elle.

9. Système de stimulateur cardiaque selon l'une des revendications 1 à 8, **caractérisé en ce que** l'unité de commande (10) dispose d'un moyen de guidage qui fonctionne de concert avec l'élément de retenue afin de guider l'unité de commande (10) pendant la disposition du mécanisme de retenue (12) dans une position de fixation définie par rapport au mécanisme de retenue (12).
